# EUROPEAN PATENT APPLICATION

(11) **EP 4 223 283 A1**
(43) Date of publication of application: **09.08.2023**
(21) Application number: 21875777.1
(22) Date of filing: 30.09.2021
(51) Int. Cl.: A61K 9/107, A61K 31/138, A61K 45/00, A61K 47/24, A61P 35/00

(54) **WATER-SOLUBLE COMPLEX CONTAINING BETA- BLOCKER AND LECITHIN**

(30) Priority: 30.09.2020 JP 2020165384
(71) Applicant: Nissan Chemical Corporation, Tokyo 103-6119 (JP); National University Corporation Chiba University, Chiba-shi, Chiba 263-8522 (JP)
(72) Inventor: MONMA Soichi, Funabashi-shi, Chiba 274-8507 (JP); ISHII Itsuko, Chiba-shi, Chiba 260-8677 (JP)
(74) Representative: dompatent von Kreisler Selting Werner - Partnerschaft von Patent- und Rechtsanwälten mbB
(86) International application number: PCT/JP2021/036088
(87) International publication number: WO 2022/071481

(57) **Abstract**

An object of the present invention is to provide an external pharmaceutical composition that imparts light stability to a β-blocker (particularly propranolol hydrochloride) and is excellent in the balance between transdermal absorbability and skin retention, and the present invention provides a complex composed of a water-soluble β-blocker and lecithin, a method for producing the complex, an external pharmaceutical composition containing the complex, and the like. The complex or a dispersion in oil containing the complex is applied to an existing pharmaceutical external preparation formulation (for example, W/O type cream), and a complex containing a β-blocker is allowed to be present in the O phase, thereby avoiding contact with water or dissolved oxygen and reducing the risk of photolysis.

## Description

### Technical Field

The present invention relates to a complex containing a water-soluble β-blocker and lecithin, a method for producing the complex, an external pharmaceutical composition containing the complex, and the like.

### Background Art

Infantile hemangiomas are defined by clinical practice guidelines for hemangiomas and vascular malformations as lesions of this nature with tumorous proliferation of abnormal vascular endothelial cells. It is known that infantile hemangioma is the most frequent tumor occurring in infants, the incidence of all infants is 0.8%, and the incidence of female infants is approximately three times higher than that of male infants. In 40% of patients, no hemangiomas are present at birth, and cell proliferation begins and rapidly increases within the first few weeks of life.
Thereafter, the hemangiomas increase and rise up to 6 to 20 months after birth, and then slowly regress from 1 year and 6 months to 5 years of age. Generation sites are head and neck 60%, trunk 25%, and limbs 15%. There are many cases where spontaneous regression occurs over time, but after increase, there is a problem in terms of appearance such as cutaneous atrophic scar, wrinkle scar, vasodilation, skin depigmentation, pigmentation, and deformation due to residual subcutaneous tumor.

Hitherto, a method of waiting for spontaneous regression by follow-up, surgical treatment, laser treatment, steroid local injection or treatment by internal administration has been mainly performed, but in recent years, a remarkable tumor reduction effect by internal administration of a β-blocker (for example, propranolol) has been reported, and in Japan, Hemangiol (registered trademark) syrup has been approved for adaptation to infantile hemangioma.

However, the internal administration of the β-blocker has a decrease in blood pressure, hypoglycemia, bradycardia and the like as side effects due to systemic administration, and a safety problem remains as a problem (Non Patent Literature 1).

Therefore, a treatment by locally administering a β-blocker such as propranolol or timolol as an external pharmaceutical composition has been attempted, and a report has been made that the effect has been recognized (Non Patent Literatures 2 and 3).

### Citation List

### Non Patent Literature

Non Patent Literature 1: Hemangiol (registered trademark) Syrup for Pediatric 0.375% Pharmaceutical Interview Form, Maruho Co., Ltd., September 2016 (Third Edition)
Non Patent Literature 2: Drug Delivery System Vol.1, No.1, 1986, p26
Non Patent Literature 3: The Journal of Bioscience and Medicine 7, 2 (2017)

### Summary of Invention

### Technical Problem

For example, propranolol has been reported for photolysis (Photochem, Photobiol. Sci., 2002, 1, 136-140, Environ. Sci. Technol., 2007, 41, 803-810), and it is known that decomposition particularly proceeds in an aqueous solution. As described above, some β-blockers have a risk of photolysis. Therefore, caution is required in the preparation of an external pharmaceutical composition containing a β-blocker, which has a higher risk of exposure to natural light or the like as compared with oral preparations.

In addition, as external pharmaceutical compositions containing a β-blocker, an aqueous gel agent, a cream, an ointment, and the like have been reported (WO 2009/050567 A, WO 2010/122442 A), but these do not satisfy both transdermal absorbability and skin retention. In particular, when an external pharmaceutical composition containing a β-blocker is applied to hemangiomas such as infantile hemangiomas, it is necessary to consider balance between transdermal absorbability and skin retention.

Therefore, an object of the present invention is to provide a composition containing a β-blocker, which has a low risk of photolysis of the β-blocker and is excellent in the balance between transdermal absorbability and skin retention.

### Solution to Problem

The present inventors have conceived an idea that if a β-blocker (propranolol) is used as a complex with something and can be always allowed to present in oil, there is almost no contact with dissolved oxygen or water even when exposed to natural light, and thus there is a possibility that the risk of photolysis is suppressed.

Then, as a result of intensive studies based on this idea, it has been found that lecithin, which is a biocompatible material, can easily form a complex with a β-blocker (propranolol), and these complexes can be stably dispersed in oil to form a complex dispersion.

External preparations such as cream preparations prepared using these complex dispersions suppress photolysis and are excellent in the balance between transdermal absorbability and skin retention, and thus have completed the present invention.

Therefore, the present invention provides the following.
[1] A complex comprising a water-soluble β-blocker and lecithin.
[2] The complex according to [1], consisting of a water-soluble β-blocker and lecithin.
[3] The complex according to [1] or [2], wherein the water-soluble β-blocker contains at least one selected from propranolol, timolol, acebutolol, atenolol, carteolol, nadolol, metoprolol, and salts thereof.
[4] The complex according to any one of [1] to [3], wherein the water-soluble β-blocker is propranolol or a salt thereof.
[5] The complex according to any one of [1] to [4], wherein the lecithin is soy lecithin.
[6] The complex according to any one of [1] to [5], wherein the soy lecithin contains 90 wt% or more of phosphatidylcholine.
[7] The complex according to any one of [1] to [6], wherein the complex is a reverse micelle, an S/O type complex, or a mixture thereof.
[8] A complex dispersion comprising the complex according to any one of [1] to [7] in an oily base material.
[9] A method for producing the complex according to any one of [1] to [7] or the complex dispersion according to [8], comprising adding a water-soluble β-blocker aqueous solution to an oily base material obtained by heating and dissolving lecithin, and dehydrating the mixture under reduced pressure.
[10] A method for producing the complex according to any one of [1] to [7] or the complex dispersion according to [8], comprising adding an oily base material to a gel-like substance obtained by kneading a water-soluble β-blocker aqueous solution and lecithin, and dehydrating the mixture under reduced pressure under heating conditions.
[11] The production method according to [9] or [10], wherein a weight ratio of lecithin/oily base material/water/β-blocker is 2/10/4/1.
[12] The method for producing a complex according to any one of [1] to [7], wherein a gel-like substance obtained by kneading a water-soluble β-blocker aqueous solution and lecithin or an aqueous dispersion of the gel-like substance is dried.
[13] The production method according to [12], wherein a weight ratio of water to lecithin at the time of kneading is 1: 1 to 5: 1.
[14] The production method according to [12], wherein a weight ratio of water to lecithin at the time of kneading is 2: 1.
[15] An external pharmaceutical composition comprising the complex according to any one of [1] to [7] or the complex dispersion according to [8].
[16] The external pharmaceutical composition according to [15], which is a W/O type cream.
[17] The external pharmaceutical composition according to [15] or [16] for treating or preventing infantile hemangioma.

### Advantageous Effects of Invention

It is considered that a preparation prepared using a dispersion in oil of a complex composed of a β-blocker (propranolol) and lecithin can be expected as an external therapeutic agent for infantile hemangioma because the risk of photolysis of the β-blocker is low and the balance between transdermal absorbability and skin retention is excellent.

On the other hand, the particles in the complex dispersion provided as one embodiment of the present invention are monodisperse, and can be produced with good reproducibility. These utilize the high affinity between a water-soluble β-blocker and lecithin, and can be easily produced using existing production facilities.

### Brief Description of Drawings

Fig. 1 illustrates a particle size distribution pattern by scattering intensity of a complex dispersion prepared in Example 1 in Test Example 1.
Fig. 2 illustrates a particle size distribution pattern by scattering intensity of a complex dispersion prepared in Example 2 in Test Example 1.
Fig. 3 illustrates a particle size distribution pattern by scattering intensity of an aqueous dispersion of a gel-like substance prepared in Example 3 in Test Example 1.
Fig. 4 illustrates a particle size distribution pattern by scattering intensity of a complex dispersion prepared in Example 3 in Test Example 1.
Fig. 5 illustrates a particle size distribution pattern by scattering intensity of a complex dispersion prepared in Example 4 in Test Example 1.
Fig. 6 illustrates results of evaluating transdermal absorbability of a formulation of Comparative Example 1 and formulations of Examples 5 to 7 in Test Example 2.
Fig. 7 illustrates results of evaluating transdermal absorbability of the formulation of Comparative Example 1 and formulations of Examples 8 to 10 in Test Example 2.
Fig. 8 illustrates results of evaluating transdermal absorbability of formulations of Comparative Examples 1, 3 and 4 and the formulation of Example 9 in Test Example 2.
Fig. 9 illustrates color comparison by exposure to light of formulations of Comparative Examples 1, 2, and 4 and the formulations of Examples 5, 6, 7, and 9 in Test Example 3. (a) is an appearance photograph of the formulations before the test, and (b) is appearance photographs of the formulations after the test.
Fig. 10 illustrates a comparison of HPLC charts of the formulations of Comparative Examples 1 and 2 and the formulation of Example 6 after exposure to light in Test Example 3.

### Description of Embodiments

### <Complex or complex dispersion>

The present invention provides a complex containing a water-soluble β-blocker and lecithin (hereinafter, also referred to as "β-blocker/lecithin complex") or a dispersion containing the complex in an oily base material.

The "complex" of the present invention means a so-called reverse micelle in which a water-soluble β-blocker and lecithin interact with each other by a non-covalent bond, and these molecules are self-assembled into particles, a particulate S/O type complex in which the water-soluble β-blocker is partially or entirely coated with lecithin, or a mixture of the reverse micelle and the S/O type complex. The average particle size when the complex containing the water-soluble β-blocker and lecithin of the present invention is dispersed in an oily base material is, for example, approximately 1 to approximately 50 nm, preferably approximately 2 to approximately 30 nm, and more preferably approximately 3 to approximately 20 nm.

In the present invention, the numerical range indicated using "to" indicates a range including the numerical values described before and after "to" as the minimum value and the maximum value, respectively.

The "water-soluble β-blocker" used in the present invention is not particularly limited as long as it is a β-blocker soluble in water. The "soluble in water" means that the amount of water required to dissolve 1 g or 1 mL of the water-soluble active ingredient is less than 100 mL to the extent that it dissolves within 30 minutes, for example, when vigorously shaken at 20 ± 5°C every 5 minutes for 30 seconds. Examples of such a water-soluble β-blocker include "sparingly soluble", "soluble", "easily soluble", or "extremely soluble" defined in the Japanese Pharmacopoeia.

Examples of the water-soluble β-blocker include, but are not limited to, propranolol, timolol, alprenolol, bupranolol, bufetrol, oxprenolol, atenolol, bisoprolol, betaxolol, bevantolol, metoprolol, acebutolol, celiprolol, nipradilol, tilisolol, nadolol, indenolol, carteolol, pindolol, bunitrolol, landiolol, esmolol, arotinolol, carvedilol, labetalol, amosulalol, and salts thereof, and these may be used alone or in combination of two or more thereof.

In one embodiment of the present invention, the water-soluble β-blocker contains at least one selected from propranolol, timolol, acebutolol, atenolol, carteolol, nadolol, metoprolol, and salts thereof.

In one embodiment of the present invention, the water-soluble β-blocker is propranolol or a salt thereof.

In one embodiment of the present invention, the water-soluble β-blocker is propranolol hydrochloride.

The content of the water-soluble β-blocker is not particularly limited as long as the object of the present invention can be achieved, but is, for example, approximately 10 to approximately 50 wt%, and preferably approximately 20 to approximately 40 wt%, based on the total amount of the complex of the present invention.

The "lecithin" used in the present invention is not particularly limited as long as it is lecithin, and may be naturally derived (for example, soy lecithin, egg yolk lecithin, or the like) or synthetically derived. Examples of the lecithin include those containing phosphatidylcholine, phosphatidylethanolamine, phosphatidylinositol, phosphatidylserine, sphingomyelin, and the like.

In one embodiment of the present invention, the lecithin contains phosphatidylcholine.

In one embodiment of the present invention, lecithin contains at least one of phosphatidylcholine represented by formula I: 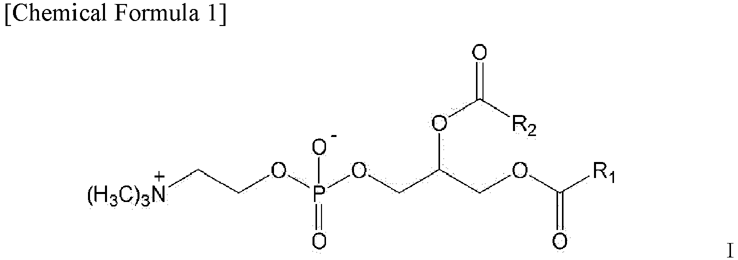 wherein:
R₁C(=O)- and R₂C(=O)- groups are independently of one another a residue (acyl group) of a saturated or unsaturated fatty acid having 2 to 22 carbon atoms, preferably a residue of a saturated or unsaturated fatty acid having 12 to 20 carbon atoms, more preferably a residue of a saturated or unsaturated fatty acid having 16 to 18 carbon atoms, and particularly preferably independently of one another a myristoyl group, a palmitoyl group, a stearoyl group, an oleoyl group, or a linoleoyl group.

In one embodiment of the present invention, lecithin contains approximately 80 wt% or more, preferably approximately 90 wt% or more, and more preferably 94 wt% or more of phosphatidylcholine (preferably phosphatidylcholine represented by formula I).

In one embodiment of the present invention, the lecithin is soy lecithin. The soy lecithin may be, for example, those appropriately available from reagent supply companies such as LIPOID AG and NOF Corporation.

The content of lecithin is not particularly limited as long as the object of the present invention can be achieved, but is, for example, approximately 40 to approximately 90 wt%, and preferably approximately 50 to approximately 80 wt%, based on the total amount of the complex of the present invention.

Alternatively, the weight ratio of the water-soluble β-blocker to lecithin contained in the complex of the present invention is not particularly limited as long as the object of the present invention can be achieved, but is, for example, approximately 1: 1 to approximately 1: 10, and preferably approximately 1: 1 to approximately 1: 3.

The "dispersion" or "complex dispersion" of the present invention means a dispersion containing a β-blocker/lecithin complex in an oily base material.

The "oily base material" used in the present invention is, for example, a substance known as a base material for pharmaceuticals, quasi-pharmaceutical products, cosmetics, nutritional supplements, or agricultural chemicals, and is not particularly limited as long as it dissolves lecithin.

Examples of the oily base material include, but are not limited to, fatty acid esters having 8 to 20 carbon atoms such as cetyl 2-ethylhexanoate, isopropyl myristate (IPM), and isopropyl palmitate (IPP), terpenes such as squalane and squalene, vegetable fats and oils such as soybean oil, jojoba oil, almond oil, and olive oil, animal fats and oils such as horse oil, synthetic oils such as silicone oil, and mineral oils such as liquid paraffin and white petrolatum, and these may be used alone or in combination of two or more thereof.

In one embodiment of the present invention, the oily base material is fatty acid esters having 10 to 16 carbon atoms, vegetable fats and oils, or mineral oils, and is preferably isopropyl myristate (IPM), soybean oil, or liquid paraffin.

### <Method for producing complex or complex dispersion>

The β-blocker/lecithin complex of the present invention or a dispersion in oil containing the complex can be produced by several methods. This is considered to be due to the high stability of the intermediate formed due to the high affinity between the water-soluble β-blocker and lecithin. The production method is roughly divided into an in-liquid drying method in which a dispersion containing a complex is produced at once in a system and a method in which the dispersion is once dehydrated via a gel-like substance.

### In-liquid drying method

This method is a method in which lecithin is heated and dissolved in an oily base material, a water-soluble β-blocker aqueous solution is added thereto and stirred, and then the mixture is dehydrated under reduced pressure to produce a transparent dispersion containing a complex at once.

The feature of this method is that a dispersion having a controlled particle size can be produced only by adding a water-soluble β-blocker aqueous solution to lecithin dissolved in an oily base material as described above, stirring the mixture, and then dehydrating the mixture without using a special shearing device. In addition, when the ratio of lecithin and the water-soluble β-blocker is appropriately set at the time of forming a complex, it is possible to produce a dispersion with high reproducibility by a single operation regardless of its scale.

The amount of the oily base material is not particularly limited, and is appropriately set according to the content in the final product. For example, an oily base material is used in an amount of approximately 3 to approximately 30 times, and preferably approximately 5 to approximately 10 times the weight of lecithin used. By using the oily base material in an amount of 3 times, and preferably 5 times or more, formation of coarse particles is suppressed, and a transparent dispersion is obtained.

The β-blocker aqueous solution means an aqueous solution in which a water-soluble β-blocker is dissolved. The β-blocker aqueous solution may further contain a pH adjusting agent or the like as long as the object of the present invention can be achieved. The β-blocker aqueous solution can be prepared by a known method. The concentration of the water-soluble β-blocker in the β-blocker aqueous solution is not particularly limited, and can be appropriately adjusted according to the solubility of the β-blocker to be used, the desired concentration and content, and the like.

When the β-blocker aqueous solution is added to the oily base material in which lecithin is dissolved, the oily base material may be once returned to around room temperature, or the β-blocker aqueous solution can be added under heating conditions as long as it is within a range in which rapid evaporation of moisture can be suppressed.

The dehydration step is performed by heating and decompressing the oily base material at a temperature equal to or lower than the boiling point of the oily base material. The heating temperature is not particularly limited, and varies depending on the oily base material to be used, but the heating is performed at, for example, 50°C to 80°C, and preferably 60°C to 75°C. Examples of the embodiment include a method of using a decompression device such as an aspirator or a vacuum pump while heating and stirring, a method of using an evaporator, and the like.

### Production method of dehydrating via gel-like substance

On the other hand, a complex or a dispersion containing the complex can be produced via a gel-like substance. The gel-like substance used in the present invention is composed of only a β-blocker aqueous solution and lecithin, and is in a gel to semisolid state in which the β-blocker aqueous solution is contained in lecithin.

Further, the methods via a gel-like substance are classified into two types depending on its drying mode. The first is a method in which a gel-like substance or an aqueous dispersion thereof is dried to form a complex of a β-blocker and lecithin, and the second is a method in which an oily base material is added to a gel-like substance and dried in a liquid to form a complex dispersion.

The gel-like substance can be obtained by kneading a water-soluble β-blocker, water, and lecithin (preferably, a β-blocker aqueous solution and lecithin). The kneading step can be performed by a known method, and can be performed until a uniform gel-like substance is formed. Instruments and devices used for kneading are not particularly limited as long as they can be usually used by those skilled in the art. Examples of the instrument/machine used for kneading include a spatula, a mortar, a stirrer, a mechanical stirrer, a mixer, a rotation-revolution mixer, a multi-bead shocker, and the like. The kneading can be performed, for example, at room temperature until a gel-like substance uniform in appearance is obtained after mixing the water-soluble β-blocker, water, and lecithin (preferably, after mixing the β-blocker aqueous solution and lecithin). In addition, operability of kneading may be improved by sufficiently immersing lecithin in a β-blocker aqueous solution before kneading.

The weight ratio of water to lecithin is not particularly limited, but is, for example, approximately 5: 1 to approximately 1: 1, preferably approximately 3: 1 to approximately 2: 1, and more preferably approximately 2: 1.

Next, a complex or a complex dispersion can be produced by subjecting the gel-like substance thus obtained to a drying step.

### (i) Method of directly freeze-drying gel-like substance

Freezing of the gel-like substance may be, for example, a method usually performed by those skilled in the art. For example, a dry bath prepared by adding dry ice to liquid nitrogen or a solvent such as acetone or methanol can be used. The frozen gel-like substance is freeze-dried for approximately 12 hours to approximately 24 hours to produce a complex, and an oily base material is added to the obtained complex, and the mixture is sufficiently stirred until transparently dispersed, whereby a complex dispersion can be produced.

### (ii) Method of drying aqueous dispersion of gel-like substance

The method for obtaining the aqueous dispersion of the gel-like substance may be, for example, a method usually performed by those skilled in the art, and examples thereof include a method in which water is added to the gel-like substance and the mixture is stirred using a stirrer, a mechanical stirrer, or the like until it becomes uniform. The weight ratio of the gel-like substance to water is not particularly limited, but is, for example, approximately 1: 1 to approximately 1: 20, preferably approximately 1: 5 to approximately 1: 10, and more preferably approximately 1: 9.

The obtained aqueous dispersion is freeze-dried in the same manner as in (i), or dried using a spray dryer (or a spray freeze-dryer), whereby a complex can be produced. In addition, an oily base material is added to the obtained complex, and the mixture is sufficiently stirred until transparently dispersed, whereby a complex dispersion can be produced. When the aqueous dispersion is used, not only operability of the gel-like substance is improved and movement between the steps is facilitated, but also the range of drying methods that can be used may be expanded.

### (iii) Method of adding oily base material to gel-like substance and drying in liquid

It is also possible to produce a complex dispersion by adding an oily base material to a gel-like substance and dehydrating the mixture under reduced pressure under heating conditions. The oily base material, the dehydration method, and the like to be used are the same as those in the "in-liquid drying method" described above.

The obtained dispersion containing the β-blocker/lecithin complex is transparent and can have high dispersion stability. Furthermore, when the complex is dispersed in fatty acid esters, addition of a certain amount of soybean oil may particularly improve light stability and dispersion stability. The dispersion thus obtained can be appropriately applied to various existing preparation formulations.

### Application of complex containing β-blocker and lecithin

The complex of the β-blocker and lecithin of the present invention or a dispersion containing the same can be used for diseases, disorders and symptoms that can be treated or prevented by the β-blocker, and the like. Examples of the diseases, disorders, and symptoms that can be treated or prevented by the β-blocker include, but are not limited to, hypertension including essential hypertension, angina, arrhythmia, migraine, hemangioma including infantile hemangioma, and the like.

In one embodiment of the present invention, there is provided the β-blocker/lecithin complex of the present invention or the dispersion containing the same for using in the treatment or prevention of hypertension including essential hypertension, angina, arrhythmia, migraine, or hemangioma including infantile hemangioma.

In one embodiment of the present invention, there is provided the β-blocker/lecithin complex of the present invention or the dispersion containing the same for using in the treatment or prevention of infantile hemangioma.

In one embodiment of the present invention, there is provided a use of the β-blocker/lecithin complex of the present invention or the dispersion containing the same for preparing a medicament for treating or preventing hypertension including essential hypertension, angina, arrhythmia, migraine, or hemangioma including infantile hemangioma.

In one embodiment of the present invention, there is provided a use of the β-blocker/lecithin complex of the present invention or the dispersion containing the same for preparing a medicament for treating or preventing infantile hemangioma is provided.

In one embodiment of the present invention, there is provided a method for treating or preventing hypertension including essential hypertension, angina, arrhythmia, migraine, or hemangioma including infantile hemangioma, comprising administering an effective amount of the β-blocker/lecithin complex of the present invention or the dispersion containing the same.

In one embodiment of the present invention, there is provided a method for treating or preventing infantile hemangioma, comprising administering an effective amount of the β-blocker/lecithin complex of the present invention or the dispersion comprising the same.

### External pharmaceutical composition

In one embodiment of the present invention, an external pharmaceutical composition containing the β-blocker/lecithin complex of the present invention or the dispersion containing the same is provided.

In addition to the β-blocker/lecithin complex of the present invention or the dispersion containing the same, the external pharmaceutical composition of the present invention may contain additives that are acceptable in the field of pharmaceuticals and cosmetics, such as excipients, lubricants, binders, disintegrants, emulsifiers, stabilizers, flavoring agents, diluents, gelling agents, and water, as necessary.

The content of the β-blocker in the external pharmaceutical composition of the present invention is not particularly limited, and can be appropriately adjusted according to the purpose.

The external pharmaceutical composition of the present invention may be formulated into a cream (a W/O type cream, an O/W type cream, or the like), a gel agent (for example, an oil gel agent or the like), an emulsion, a suspending agent, a patch (a tape agent, a cataplasm agent, or the like), or the like together with the above additives as necessary with reference to a known method or the like.

For example, in the case of a W/O type cream, a mineral oil such as white petrolatum and an emulsion stabilization aid such as stearyl alcohol are heated and dissolved to form an oil phase. Next, a lipophilic nonionic surfactant and a heated aqueous phase are added dropwise to the oil phase and sufficiently emulsified. When the emulsification is completed, a dispersion containing the β-blocker/lecithin complex of the present invention may be added, stirred until it becomes uniform in a heated state, and allowed to cool to obtain a W/O type cream.

Examples of the lipophilic nonionic surfactant include sorbitan monooleate, sorbitan sesquioleate, polyoxyethylene-hydrogenated castor oil, polyoxyethylene (2) oleyl ether, and the like.

In the case of an O/W type cream, for example, an O/W type cream may be obtained by heating purified water containing a surfactant having a high HLB value and various additives (for example, preservatives such as ethyl parahydroxybenzoate and propyl parahydroxybenzoate, solubilizing agents such as propylene glycol, and the like), dropping the oil phase similarly heated, stirring the mixture in a heated state until it becomes uniform, and allowing the mixture to cool.

In the case of an oil gel agent, for example, dextrin palmitate, which is an oil thickening/gelling agent, may be added to a mineral oil such as liquid paraffin, and heated and dissolved, then a dispersion containing the β-blocker/lecithin complex of the present invention may be gradually added and stirred, stirring may be stopped when the dispersion becomes uniform, and then the dispersion may be allowed to cool to room temperature to obtain an oil gel agent.

In one embodiment of the present invention, the external pharmaceutical composition of the present invention which is a W/O type cream is provided.

By using a W/O type cream, it is possible to impart light stability while maintaining high transdermal absorbability and retention. In addition, in these dosage forms, drying of the agent after application is suppressed, so that there is no precipitation of a gelling agent that causes a problem when an aqueous gel agent is applied. Therefore, itchy sensation of the affected part associated with the precipitation of the gelling agent is reduced, and the risk of scratching the affected part is reduced.

In one embodiment of the present invention, there is provided the external pharmaceutical composition of the present invention for treating or preventing hypertension including essential hypertension, angina, arrhythmia, migraine, or hemangioma including infantile hemangioma.

In one embodiment of the present invention, the external pharmaceutical composition of the present invention for treating or preventing infantile hemangioma is provided.

### Examples

The present invention will be described in more detail with reference to the following examples, but the present invention is not limited thereto.

The following reagents were used.
· Propranolol hydrochloride: manufactured by Tokyo Chemical Industry Co., Ltd.
· Soy lecithin (containing 94% or more of phosphatidylcholine): Trade name "Phospholipon (registered trademark) 90G", manufactured by Lipoid AG
· Isopropyl myristate: Trade name "EXCEPARL IPM", manufactured by Kao Corporation
· Japanese Pharmacopoeia white petrolatum: Kozakai Pharmaceutical Co., Ltd.
· Japanese Pharmacopoeia stearyl alcohol: manufactured by Kao Corporation
· Polyoxyethylene (2) oleyl ether: Trade name "NIKKOL BO-2V", manufactured by Nikko Chemicals Co., Ltd.
· Japanese Pharmacopoeia soybean oil: manufactured by KANEDA Co., Ltd.
· Sodium lauryl sulfate: Trade name "EMAL OS", manufactured by Kao Corporation
· Japanese Pharmacopoeia propylene glycol: manufactured by Maruishi Pharmaceutical Co.,Ltd.
· Purified liquid paraffin: Trade name "MORESCO Bioless (U-6)", manufactured by MORESCO Corporation
· Dextrin palmitate: Trade name "Rheopearl KL-2", manufactured by Chiba Flour Milling Co., Ltd.
· Sucrose laurate: Trade name "L-195", manufactured by Mitsubishi-Chemical Foods Corporation
· Sucrose erucate: Trade name "ER290", manufactured by Mitsubishi-Chemical Foods Corporation

As an emulsifier used in the emulsification step, CLEARMIX manufactured by M Technique Co., Ltd. was used.

Preparation of dispersion containing propranolol hydrochloride-soy lecithin complex

### [Example 1: In-liquid drying method]

Purified water (2.0 g) was added to propranolol hydrochloride (0.5 g), and completely dissolved in a hot water bath at 50°C to prepare an aqueous solution of propranolol hydrochloride. Separately, soy lecithin (1.0 g) and isopropyl myristate (5.0 g) were added to an eggplant flask, and completely dissolved in a hot water bath at 75°C, then the aqueous solution of propranolol hydrochloride prepared was added dropwise, and the mixture was stirred for approximately 10 minutes.

The obtained suspension was dried under reduced pressure at 75°C for 1 hour and then allowed to cool to obtain a desired dispersion.

### [Example 2: Method of freeze-drying gel-like substance]

Purified water (2.0 g) was added to propranolol hydrochloride (0.4 g), and completely dissolved in a hot water bath at 50°C to prepare an aqueous solution of propranolol hydrochloride. The aqueous solution of propranolol hydrochloride prepared was added to soy lecithin (1.0 g), and the mixture was sealed and immersed at room temperature for approximately 3 hours. The mixture was kneaded with a spatula until it became uniform to obtain a gel-like substance. The obtained gel-like substance was frozen with dry ice-acetone and dried in a freeze-dryer for approximately 19 hours. Isopropyl myristate (10 g) was added to the obtained dried product, and the mixture was stirred until it was uniformly dispersed to obtain a desired dispersion.

### [Example 3: Method of drying gel-like substance as aqueous dispersion]

Purified water (30.6 g) was added to a gel-like substance prepared in the same manner as in Example 2, and the mixture was stirred until it was uniformly dispersed to obtain an aqueous dispersion of a gel-like substance. Subsequently, the obtained aqueous dispersion (17.0 g) was frozen in dry ice-acetone and dried in a freeze-dryer for approximately 19 hours. Isopropyl myristate (5.0 g) was added to the dried product thus obtained, and the mixture was stirred until it was uniformly dispersed to obtain a desired dispersion.

### [Example 4: Drying in liquid via gel-like substance]

Isopropyl myristate (10 g) was added to a gel-like substance prepared in the same manner as Example 2, and the mixture was dried under reduced pressure in a warm bath at 75°C for 30 minutes and then allowed to cool to obtain a desired dispersion.

All of the complex dispersions prepared in Examples 1 to 4 were transparent, and their particle sizes were also equivalent (see Test Example 1).

Preparation of external pharmaceutical composition containing propranolol hydrochloride-soy lecithin complex dispersion

### [Examples 5 to 10: Preparation of W/O type creams]

Japanese Pharmacopoeia white petrolatum, Japanese Pharmacopoeia stearyl alcohol, and polyoxyethylene (2) oleyl ether were each weighed in a container, and stirred in a hot water bath at 75°C until they became uniform. The mixture was attached to an emulsifier, and purified water was added dropwise thereto while stirring at 75°C and 4,500 rpm. After approximately 10 minutes, when the whole mixture became uniform, a complex dispersion prepared in the same manner as in Example 1 and Japanese Pharmacopoeia soybean oil were added thereto, and the mixture was further heated and stirred for 10 minutes. The container was pulled up from the hot water bath, and the mixture was stirred until the temperature reached room temperature while being allowed to cool, thereby obtaining a desired W/O type cream.

Table 1 and Table 2 show the amounts (weight ratios) of the reagents used for preparation of the creams of Examples 5 to 7 and Examples 8 to 10, respectively.

**[Table 1]**

| Reagent | Example 5 | Example 6 | Example 7 |
|---|---|---|---|
| Japanese Pharmacopoeia white petrolatum | 36.0 g (36 wt%) | 31.0 g (31 wt%) | 31.0 g (31 wt%) |
| Japanese Pharmacopoeia stearyl alcohol | 16.0 g (16 wt%) | 21.0 g (21 wt%) | 16.0 g (16 wt%) |
| Polyoxyethylene (2) oleyl ether | 5.0 g (5 wt%) | 5.0 g (5 wt%) | 5.0 g (5 wt%) |
| Purified water | 20.0 g (20 wt%) | 20.0 g (20 wt%) | 25.0 g (25 wt%) |
| Propranolol hydrochloride | 1.0 g (1 wt%) | 1.0 g (1 wt%) | 1.0 g (1 wt%) |
| Soy lecithin | 2.0 g (2 wt%) | 2.0 g (2 wt%) | 2.0 g (2 wt%) |
| Isopropyl myristate | 10 g (10 wt%) | 10 g (10 wt%) | 10 g (10 wt%) |
| Japanese Pharmacopoeia soybean oil | 10 g (10 wt%) | 10 g (10 wt%) | 10 g (10 wt%) |
| Total amount | 100 g | 100 g | 100 g |
| | (100 wt%) | (100 wt%) | (100 wt%) |

**[Table 2]**

| Reagent | Example 8 | Example 9 | Example 10 |
|---|---|---|---|
| Japanese Pharmacopoeia white petrolatum | 28.0 g (28 wt%) | 24.0 g (24 wt%) | 20.0 g (20 wt%) |
| Japanese Pharmacopoeia stearyl alcohol | 16.0 g (16 wt%) | 20 g (20 wt%) | 24.0 g (16 wt%) |
| Polyoxyethylene (2) oleyl ether | 8.0 g (8 wt%) | 8.0 g (8 wt%) | 8.0 g (8 wt%) |
| Purified water | 25.0 g (25 wt%) | 25.0 g (25 wt%) | 25.0 g (25 wt%) |
| Propranolol hydrochloride | 1.0 g (1 wt%) | 1.0 g (1 wt%) | 1.0 g (1 wt%) |
| Soy lecithin | 2.0 g (2 wt%) | 2.0 g (2 wt%) | 2.0 g (2 wt%) |
| Isopropyl myristate | 10 g (10 wt%) | 10 g (10 wt%) | 10 g (10 wt%) |
| Japanese Pharmacopoeia soybean oil | 10 g (10 wt%) | 10 g (10 wt%) | 10 g (10 wt%) |
| Total amount | 100 g (100 wt%) | 100 g (100 wt%) | 100 g (100 wt%) |

### [Example 11: Preparation of O/W type cream]

Purified water [20.1 g (40.2 wt%)], sodium lauryl sulfate [0.9 g (1.8 wt%)], and Japanese Pharmacopoeia propylene glycol [2.5 g (5.0 wt%)] were each weighed in necessary amounts in a container, and stirred in a hot water bath at 75°C until the mixture became uniform to form an aqueous phase. On the other hand, Japanese Pharmacopoeia white petrolatum [10 g (20 wt%)], Japanese Pharmacopoeia stearyl alcohol [5.0 g (10 wt%)], and a complex dispersion [11.5 g (23.0 wt%)] prepared in the same manner as in Example 2 were added to another container, and stirred in a hot water bath at 75°C until the mixture became uniform to form an oil phase. The oil phase was added dropwise while the aqueous phase was heated and stirred in a hot water bath at 75°C, and stirred until it became uniform. The mixture was taken out from the hot water bath and allowed to cool overnight to obtain a desired O/W type cream.

### [Example 12: Preparation of oil gel agent]

Purified liquid paraffin [27.5 g (55.0 wt%)] was weighed in a container, and dextrin palmitate [2.5 g (5.0 wt%)] was added little by little. The mixture was sufficiently stirred at room temperature, and then completely dissolved in a warm bath at 75°C. A complex dispersion [20.0 g (40.0 wt%)] prepared in the same manner as in Example 2 was added to the resulting mixed liquid, and the mixture was stirred at 75°C until it became uniform. The stirring was stopped at 75°C, and the mixture was placed in a container and cooled to room temperature over approximately 12 hours to obtain a desired oil gel agent.

### [Comparative Example 1: Preparation of gel agent containing propranolol]

Propranolol hydrochloride (0.2 g) was weighed into a container, and phosphate buffered saline (20 mL) was added thereto, then the mixture was heated to 70°C while stirring. Hydroxypropylmethyl cellulose [0.9 g, (4.5% (w/v))] was added thereto and suspended, then cooled and stirred until transparent to obtain a gel agent of Comparative Example 1.

### [Comparative Example 2: Preparation of W/O type cream containing propranolol-1]

Japanese Pharmacopoeia white petrolatum [40g, (40 wt%)], Japanese Pharmacopoeia stearyl alcohol 20 g, (20 wt%)], and polyoxyethylene (2) oleyl ether [5.0 g, (5 wt%)] were each weighed in a container, and stirred in a hot water bath at 75°C until they became uniform to form an oil phase. On the other hand, propranolol hydrochloride [1.0 g (1.0 wt%)] and purified water [34.0 g (34 wt%)] were weighed in required amounts in different containers, and completely dissolved in a hot water bath at 75°C to form an aqueous phase. The oil phase was attached to an emulsifier, and the aqueous phase was added dropwise while stirring at 75°C and 4,500 rpm, and the mixture was emulsified for approximately 10 minutes. The container was pulled up from the hot water bath, and the mixture was stirred until the temperature reached room temperature while being allowed to cool, thereby obtaining a desired W/O type cream.

### [Comparative Examples 3 to 4: Preparation of W/O type creams containing propranolol-2]

Japanese Pharmacopoeia white petrolatum, Japanese Pharmacopoeia stearyl alcohol, and polyoxyethylene (2) oleyl ether were each weighed in a container, and stirred in a hot water bath at 75°C until they became uniform. The mixture was attached to an emulsifier, and an aqueous solution of propranolol hydrochloride was added dropwise thereto while stirring the mixture at 75°C and 4,500 rpm. After approximately 10 minutes, when the whole mixture became uniform, isopropyl myristate in which soy lecithin was heated and dissolved in advance and Japanese Pharmacopoeia soybean oil were added thereto, and the mixture was further heated and stirred for 10 minutes. The container was pulled up from the hot water bath, and the mixture was stirred until the temperature reached room temperature while being allowed to cool, thereby obtaining a desired W/O type cream.

Table 3 shows the amount (weight ratio) of each reagent used for preparing the creams of Comparative Examples 3 to 4.

**[Table 3]**

| Reagent | Comparative Example 3 | Comparative Example 4 |
|---|---|---|
| Japanese Pharmacopoeia white petrolatum | 31.0 g (31 wt%) | 24.0 g (24 wt%) |
| Japanese Pharmacopoeia stearyl alcohol | 21.0 g (21 wt%) | 20.0 g (20 wt%) |
| Polyoxyethylene (2) oleyl ether | 5.0 g (5 wt%) | 8.0 g (8 wt%) |
| Purified water | 20.0 g (20 wt%) | 25.0 g (25 wt%) |
| Propranolol hydrochloride | 1.0 g (1 wt%) | 1.0 g (1 wt%) |
| Soy lecithin | 2.0 g (2 wt%) | 2.0 g (2 wt%) |
| Isopropyl myristate | 10 g (10 wt%) | 10 g (10 wt%) |
| Japanese Pharmacopoeia soybean oil | 10 g (10 wt/%) | 10 g (10 wt%) |
| Total amount | 100 g (100 wt%) | 100 g (100 wt%) |

### [Comparative Example 5: In-liquid drying method using surfactant other than soy lecithin]

In-liquid drying was performed in the same manner as in Example 1 using sucrose laurate or sucrose erucate, which is a surfactant having a low HLB value, instead of using soy lecithin, but a suspension was formed, and a transparent dispersion could not be obtained.

### [Test Example 1: Evaluation of particle size]

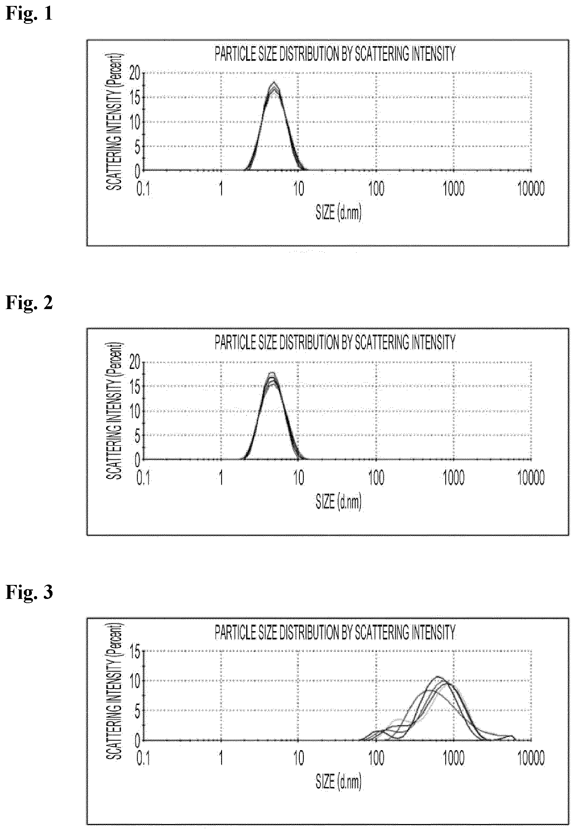

The particle size of the complex dispersion obtained in Examples 1 to 4 was measured using Zetasizer Nano ZS (manufactured by Malvern Panalytical) measured using a dynamic light scattering method (DLS). For the measurement, a glass cell was used, and physical property values [viscosity: 6.6 mPa·s (20°C), refractive index: 1.434] of EXCEPARL IPM were used. Also, the average particle size indicates the average value (nm) of the particle size distribution by scattering intensity. The results are shown in Figs. 1 to 4.

### Results

The average particle size in the complex dispersion prepared in Example 1 was 4.701 nm (PDI: 0.126) (Fig. 1).

The average particle size of the dispersion when the amount of propranolol hydrochloride was changed was as follows.

**[Table 4]**

| | Propranolol hydrochloride (g) | Z Average | PDI |
|---|---|---|---|
| 1 | 0.4 | 4.463 | 0.107 |
| 2 | 0.45 | 4.364 | 0.055 |
| 3 | 0.5 | 4.375 | 0.045 |
| 4 | 0.6 | 4.449 | 0.066 |
| 5 | 0.7 | 4.372 | 0.046 |

The average particle size in the complex dispersion prepared in Example 2 was 4.443 nm (PDI: 0.105) (Fig. 2).

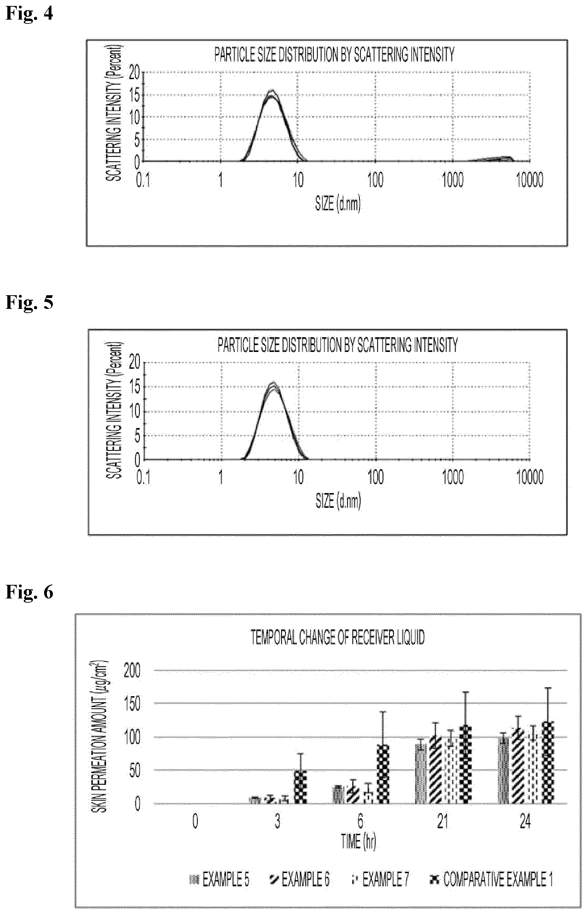

The average particle size of the aqueous dispersion of the gel-like substance obtained in Example 3 was 494.4 nm (PDI: 0.363) (Fig. 3). It was confirmed that the particles were stably dispersed without causing a change in particle size even after approximately 2 weeks. The average particle size of the finally obtained complex dispersion was 4.481 nm (PDI: 0.137) (Fig. 4).

The average particle size of the complex dispersion obtained in Example 4 was 4.478 nm (PDI: 0.106) (Fig. 5).

### [Test Example 2: Transdermal absorption test]

Skin of a hairless mouse [trade name: "Laboskin", manufactured by Hoshino Laboratory Animals, Inc.] was attached to a vertical Franz cell (10 mm in line) manufactured by Asahi Glassplant Inc. As a receiver liquid of the cell, a phosphate buffer (manufactured by FUJIFILM Wako Pure Chemical Corporation) was used.

The formulations obtained in Examples 5 to 10 and Comparative Examples 1, 3 and 4 were applied (50 mg) to the skin of a hairless mouse, and a test was performed at 37°C for 24 hours. The propranolol concentration in the receiver liquid was measured by HPLC at 3 hours and 6 hours after the start of the test, 3 hours before the end of the test, and at the end of the test. The results are shown in Figs. 6 to 8.

The measurement conditions of HPLC are as follows.
· Column used: Xbridge BEH C18 (2.5 µm, 4.6 mm Φ X 7.5 cm) manufactured by Waters Corporation
· Column temperature: 40°C
· Eluent: Water + 0.1% TFA/CH₃CN + 0.1% TFA = 80/20 → 5/95 (gradient)
· Flow rate: 0.5 mL/min
· Measurement wavelength: 290 nm

### Results

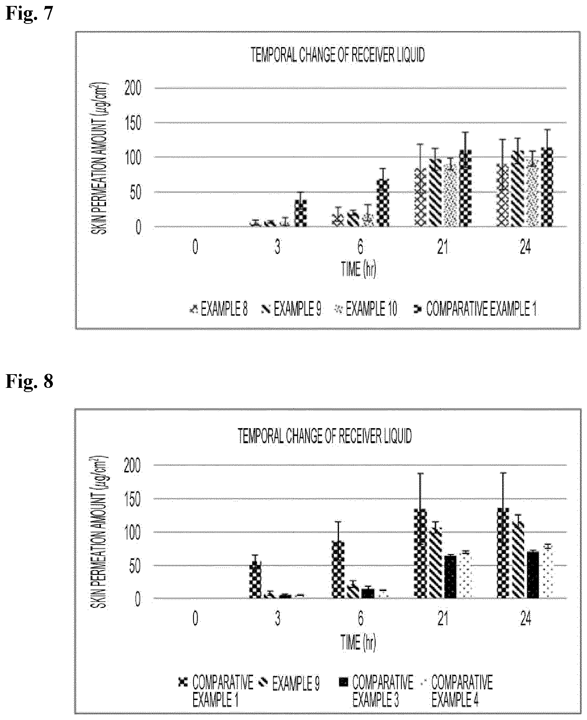

As is apparent from Figs. 6 and 7, by applying the propranolol-soy lecithin complex dispersion to the W/O type cream, the transdermal absorbability equivalent to that of the aqueous gel (Comparative Example 1) was exhibited.

As is apparent from Fig. 8, the complex-dispersed cream (Example 9) and the sample obtained by adding soy lecithin to the normal W/O type cream (Comparative Example 4) had exactly the same composition, but the complex-dispersed cream showed higher transdermal absorbability.

### [Test Example 3: Evaluation of light stability (light stability test apparatus)]

An appropriate amount of each of the formulations obtained in Examples 5, 6, 7, and 9 and Comparative Examples 1, 2, and 4 was put in a glass bottle, and color tone change due to accumulation of exposure to light was evaluated with a light stability test apparatus [model: LTL-200A-14WCD (manufactured by Nagano Science Co., Ltd.)]. For the formulations of Example 6 and Comparative Examples 1 and 2, the purity was measured by HPLC after exposure to light.

The test conditions are shown below.
Light source: D65 lamp, temperature: 25°C, relative humidity: 60% RH
Illuminance: 4.00 klx, integrated illuminance: 1200 klx·h

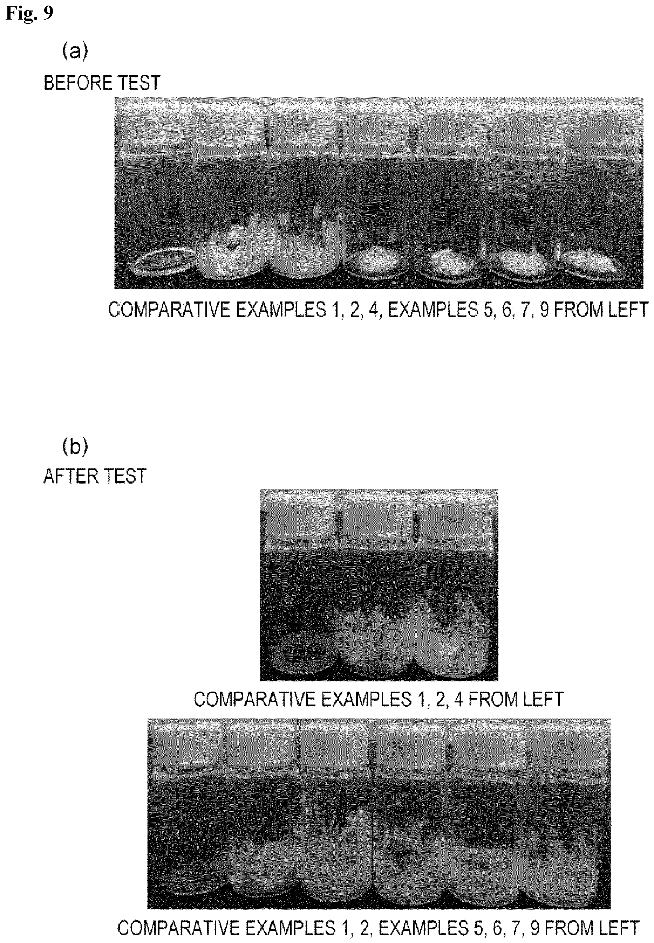

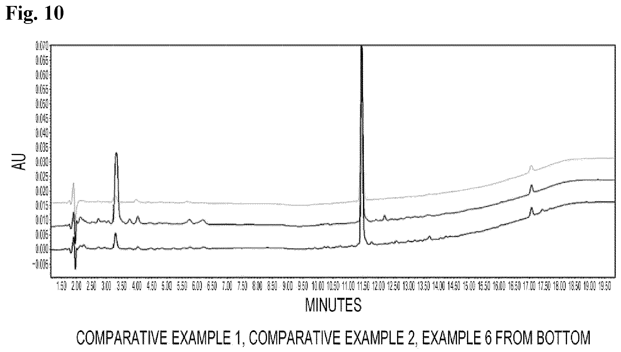

The measurement by HPLC was performed under the same conditions as in Test Example 2 except that the measurement wavelength was 254 nm. Appearance photographs of the formulations before and after exposure to light are shown in Fig. 9, and an HPLC chart obtained as a result of HPLC measurement is shown in Fig. 10.

### Results

As is apparent from Fig. 9, severe coloring was observed in the formulations of Comparative Examples 1 and 2 after the test, but coloring was suppressed in the formulations of Comparative Example 4 and Examples 5, 6, 7, and 9.

A main decomposition product of Comparative Example 2 is a peak appearing at approximately 3.3 minutes on the HPLC chart of Fig. 10, and it can be estimated that a naphthalene ring of propranolol is oxidatively cleaved from the molecular weight of LC/MS and literature information. Further, in Comparative Example 1 having strong coloration, a large number of small decomposition product peaks were formed, and it is considered that decomposition further proceeded from the oxidative cleavage product.

## Claims

1. A complex comprising a water-soluble β-blocker and lecithin.

2. The complex according to claim 1, consisting of a water-soluble β-blocker and lecithin.

3. The complex according to claim 1 or 2, wherein the water-soluble β-blocker contains at least one selected from propranolol, timolol, acebutolol, atenolol, carteolol, nadolol, metoprolol, and salts thereof.

4. The complex according to any one of claims 1 to 3, wherein the water-soluble β-blocker is propranolol or a salt thereof.

5. The complex according to any one of claims 1 to 4, wherein the lecithin is soy lecithin.

6. The complex according to any one of claims 1 to 5, wherein the soy lecithin contains 90 wt% or more of phosphatidylcholine.

7. The complex according to any one of claims 1 to 6, wherein the complex is a reverse micelle, an S/O type complex, or a mixture thereof.

8. A complex dispersion comprising the complex according to any one of claims 1 to 7 in an oily base material.

9. A method for producing the complex according to any one of claims 1 to 7 or the complex dispersion according to claim 8, comprising adding a water-soluble β-blocker aqueous solution to an oily base material obtained by heating and dissolving lecithin, and dehydrating the mixture under reduced pressure.

10. A method for producing the complex according to any one of claims 1 to 7 or the complex dispersion according to claim 8, comprising adding an oily base material to a gel-like substance obtained by kneading a water-soluble β-blocker aqueous solution and lecithin, and dehydrating the mixture under reduced pressure under heating conditions.

11. The production method according to claim 9 or 10, wherein a weight ratio of lecithin/oily base material/water/β-blocker is 2/10/4/1.

12. The method for producing the complex according to any one of claims 1 to 7, wherein a gel-like substance obtained by kneading a water-soluble β-blocker aqueous solution and lecithin or an aqueous dispersion of the gel-like substance is dried.

13. The production method according to claim 12, wherein a weight ratio of water to lecithin at the time of kneading is 1: 1 to 5: 1.

14. The production method according to claim 12, wherein a weight ratio of water to lecithin at the time of kneading is 2: 1.

15. An external pharmaceutical composition comprising the complex according to any one of claim 1 to 7 or the complex dispersion according to claim 8.

16. The external pharmaceutical composition according to claim 15, which is a W/O type cream.

17. The external pharmaceutical composition according to claim 15 or 16 for treating or preventing infantile hemangioma.
